# EUROPEAN PATENT APPLICATION

(11) **EP 2 500 029 A1**
(43) Date of publication of application: **19.09.2012**
(21) Application number: 10829941.3
(22) Date of filing: 10.11.2010
(51) Int. Cl.: A61K 38/00, A61P 1/04, A61P 1/14, A61P 43/00, C07K 5/00

(54) **THERAPEUTIC AGENT FOR GASTROINTESTINAL DISEASES**

(30) Priority: 11.11.2009 JP 2009257569
(71) Applicant: Yamaguchi University, Yamaguchi 753-8511 (JP); Santen Pharmaceutical Co., Ltd, Osaka-shi, Osaka 533-8651 (JP)
(72) Inventor: NISHIDA, Teruo, Ube-shi Yamaguchi 755-8505 (JP); INUI, Makoto, Ube-shi Yamaguchi 755-8505 (JP); MATSUURA, Kenji, Ube-shi Yamaguchi 755-8505 (JP); NAKAMURA, Masatsugu, Ikoma-shi Nara 630-0101 (JP); NAGANO, Takashi, Ikoma-shi Nara 630-0101 (JP)
(74) Representative: Müller-Boré & Partner Patentanwälte
(86) International application number: PCT/JP2010/069967
(87) International publication number: WO 2011/058982

(57) **Abstract**

The present invention provides a therapeutic agent and a prophylactic agent for a gastrointestinal disease. A peptide having an amino acid sequence represented by Ser-Ser-Ser-Arg or a pharmaceutically acceptable salt thereof promotes wound healing in a gastrointestinal mucosal tissue, and is therefore useful as a therapeutic agent or a prophylactic agent for a gastrointestinal disease. Further, when (a) a peptide having an amino acid sequence represented by Ser-Ser-Ser-Arg or a pharmaceutically acceptable salt thereof and (b) a peptide having an amino acid sequence represented by Phe-Gly-Leu-Met-NH₂ or a pharmaceutically acceptable salt thereof are administered in combination, these medicaments act synergistically and can significantly promote wound healing in a gastrointestinal mucosal tissue.

## Description

### Technical Field

The present invention relates to a therapeutic agent or a prophylactic agent for a gastrointestinal disease containing a peptide having an amino acid sequence represented by Ser-Ser-Ser-Arg or a pharmaceutically acceptable salt thereof as an active ingredient. The present invention also relates to a therapeutic agent or a prophylactic agent for a gastrointestinal disease containing (a) a peptide having an amino acid sequence represented by Ser-Ser-Ser-Arg or a pharmaceutically acceptable salt thereof and (b) a peptide having an amino acid sequence represented by Phe-Gly-Leu-Met-NH₂ or a pharmaceutically acceptable salt thereof as active ingredients.

### Background Art

The gastrointestinal tract is an organ of digestion of food and absorption of nutritive materials and vital nutrients are taken in through the mucosal tissues of the intestinal tract. However, when a gastrointestinal mucosal tissue is subjected to atrophy due to a pathological or surgical disorder or an ulcer is caused in a membrane tissue and gastrointestinal dysfunction occurs, an abnormality of intestinal mucosal permeability may sometimes be increased. Further, it is known that a gastrointestinal mucosal tissue is subjected to atrophy also due to intense radiation exposure, intestinal surgery, a drug, or stimulation that impairs cell proliferation. In this manner, if the gastrointestinal tract is subjected to invasion or atrophy, a serious gastrointestinal disorder is caused, and therefore, quick healing of the gastrointestinal tract and recovery of the gastrointestinal function are demanded.

On the other hand, a peptide having an amino acid sequence represented by Ser-Ser-Ser-Arg (hereinafter referred to as "SSSR") is a partial peptide of an insulin-like growth factor I, and a peptide having an amino acid sequence represented by Phe-Gly-Leu-Met-NH₂ (hereinafter referred to as "FGLM-NH₂") is a tetrapeptide on the C-terminal end of substance P. As the pharmaceutical use of SSSR and FGLM-NH₂ in combination, a therapeutic agent for a corneal disorder has been reported in the ophthalmological field, and also a therapeutic agent for a skin wound has been reported in the dermatological field (JP-A-2003-231695).

However, with respect to a gastrointestinal disease, there has been no study as to what sort of pharmacological effect is exhibited by single administration of SSSR or FGLM-NH₂ or combined administration of SSSR and FGLM-NH₂.

### Summary of the Invention

### Problems to be Solved

It is a very interesting subject to search for a novel medicament which is useful as a therapeutic agent and a prophylactic agent for a gastrointestinal disease such as gastrointestinal mucosal atrophy, gastrointestinal dysfunction, or a digestive membrane wound.

### Means for Solving the Problems

The present inventors found that SSSR promotes the migration of gastrointestinal mucosal tissue cells and therefore exhibits an excellent therapeutic effect on a gastrointestinal disease such as recovery from gastrointestinal damage by carrying out a pharmacological test using small intestinal mucosal tissue cells, and thus achieved the present invention. The present inventors also found that by using SSSR and FGLM-NH₂ in combination, even if the concentration of SSSR is low, these medicaments act synergistically and promote the migration of gastrointestinal mucosal tissue cells significantly.

The present invention is directed to a therapeutic agent or a prophylactic agent for a gastrointestinal disease containing SSSR or a pharmaceutically acceptable salt thereof and FGLM-NH₂ or a pharmaceutically acceptable salt thereof as active ingredients, and further is directed to a therapeutic agent or a prophylactic agent for a gastrointestinal disease containing (a) a peptide having an amino acid sequence represented by Ser-Ser-Ser-Arg or a pharmaceutically acceptable salt thereof and (b) a peptide having an amino acid sequence represented by Phe-Gly-Leu-Met-NH₂ or a pharmaceutically acceptable salt thereof as active ingredients.

In the present invention, examples of the pharmaceutically acceptable salt include hydrochlorides, sulfates, phosphates, lactates, acetates, trifluoroacetates, formates, maleates, fumarates, oxalates, methanesulfonates, and p-toluenesulfonates. More preferred are acetates and trifluoroacetates for SSSR, and hydrochlorides and trifluoroacetates for FGLM-NH₂.

In the present invention, examples of the gastrointestinal organ include organs such as esophagus, stomach, duodenum, small intestine, and large intestine, and examples of the gastrointestinal disease include various diseases such as an ulcerative or inflammatory gastrointestinal disease, a gastrointestinal disease caused by abnormal mucosal permeability, an acquired digestion and absorption disorder caused by gastrointestinal damage due to gastrointestinal resection, radiation exposure, or a drug, and a congenital digestion and absorption disorder. Here, the ulcerative gastrointestinal disease includes not only a peptic ulcer, but also erosion and an acute mucosal lesion such as an acute ulcer.

Further, the therapeutic agent or prophylactic agent for a gastrointestinal disease of the present invention is expected to be effective also in the treatment or prevention of mucosal pathology or dumping syndrome due to an inflammatory disease such as enteritis, Crohn's disease, or ulcerative colitis, and moreover, it also becomes possible to promote the cure of surgical invasion or improve the function of the gastrointestinal tract.

The therapeutic agent or prophylactic agent for a gastrointestinal disease of the present invention can be administered orally or parenterally. Examples of the dosage form include a tablet, a capsule, a granule, a powder, and an injection, and such a pharmaceutical preparation can be formulated using common techniques.

For example, an oral preparation such as a tablet, a capsule, a granule, or a powder can be prepared using an excipient such as lactose, mannitol, starch, crystalline cellulose, light anhydrous silicic acid, calcium carbonate, or calcium hydrogen phosphate; a lubricant such as stearic acid, magnesium stearate, or talc; a binder such as starch, hydroxypropyl cellulose, hydroxypropylmethyl cellulose, or polyvinylpyrrolidone; a disintegrant such as carboxymethyl cellulose, low-substituted hydroxypropyl cellulose, or calcium citrate; a coating agent such as hydroxypropylmethyl cellulose, macrogol, or a silicone resin; a stabilizer such as ethyl parahydroxybenzoate or benzyl alcohol; a corrigent such as a sweetener, a sour agent, or a flavor; or the like according to need.

Further, a parenteral preparation such as an injection can be prepared using a tonicity agent such as sodium chloride or concentrated glycerin; a buffer agent such as sodium phosphate or sodium acetate; a surfactant such as polyoxyethylene sorbitan monooleate, polyoxyl 40 stearate, or polyoxyethylene hydrogenated castor oil; a stabilizer such as sodium citrate or sodium edetate; a preservative such as benzalkonium chloride or paraben; or the like according to need.

The dose of the therapeutic agent or prophylactic agent for a gastrointestinal disease of the present invention can be appropriately selected according to symptoms, age, dosage form, and the like. For example, in the case of an oral preparation, when SSSR or a pharmaceutically acceptable salt thereof is used as a single agent, SSSR or a pharmaceutically acceptable salt thereof can be administered once or divided into several times at a dose of generally 0.01 to 5000 mg, preferably 0.01 to 1000 mg per day. Further, when SSSR or a pharmaceutically acceptable salt thereof and FGLM-NH₂ or a pharmaceutically acceptable salt thereof are used in combination, SSSR or a pharmaceutically acceptable salt thereof can be administered once or divided into several times at a dose of generally 0.0001 to 500 mg, preferably 0.001 to 100 mg per day, and FGLM-NH₂ or a pharmaceutically acceptable salt thereof can be administered once or divided into several times at a dose of generally 0.001 to 5000 mg, preferably 0.01 to 1000 mg per day.

### Advantageous Effects of the Invention

From the results of a pharmacological test, it was confirmed that a salt of SSSR has an effect of promoting the migration of gastrointestinal mucosal tissue cells. Therefore, SSSR or a pharmaceutically acceptable salt thereof is expected to exhibit an effect as a therapeutic agent or a prophylactic agent for various gastrointestinal diseases such as an ulcerative or inflammatory gastrointestinal disease, a gastrointestinal disease caused by abnormal mucosal permeability, an acquired digestion and absorption disorder caused by gastrointestinal damage due to gastrointestinal resection, radiation exposure, or a drug, and a congenital digestion and absorption disorder.

Further, when a salt of SSSR and a salt of FGLM-NH₂ are used in combination, these medicaments act synergistically, and as a result, even if SSSR is used at a low concentration at which the migration of gastrointestinal mucosal tissue cells cannot be promoted by a single agent of SSSR, these medicaments can promote the migration of gastrointestinal mucosal tissue cells significantly. Accordingly, the combined use of SSSR or a pharmaceutically acceptable salt thereof and FGLM-NH₂ or a pharmaceutically acceptable salt thereof is useful for the treatment or prevention of a gastrointestinal disease.

### Mode for Carrying Out the Invention

Hereinafter, the results of a pharmacological test and preparation examples will be described, however, these examples are for understanding the present invention better, and are not meant to limit the scope of the present invention.

### 1. Pharmacological Test (Effect on Migration of Gastrointestinal Mucosal Tissue Cells)

An effect on cell migration was studied by the following method using Caco-2 cells which are a human colon cancer cell line.

### (Experimental Method)

In a 24-well plate coated with type IV collagen, Caco-2 cells were seeded at 1 × 10⁵ cells per well and cultured to confluence in a DMEM medium containing 10% FBS. A cut was formed in the cell layer using a razor blade, and a cell-free region was created with a cotton swab. After washing with a DMEM medium containing 1% FBS, the cells were cultured for 18 hours in the same medium containing each test compound under conditions of 37°C and 5% CO₂. The effect on the migration of Caco-2 cells was studied by taking pictures before adding the test compound and at 18 hours after the addition of the test compound and comparing cell spreading areas in a fixed region at 18 hours after the addition of the test compounds. Further, Caco-2 cells cultured in the same manner in the medium without containing the test compound were used as a control.

### (Results)

The results of the case where a single agent of SSSR was used are shown in Table 1, and the results of the case where SSSR and FGLM-NH₂ were used in combination are shown in Table 2. In the tables, the migration of the Caco-2 cells is expressed as a mean and standard error calculated from the results of 8 samples for each test compound by taking the migration of the control cells as a standard (100%). Incidentally, the symbol "**" indicates a statistical significance at a P value of <0.001 compared to the control.

**[Table 1]**

| Test compound (concentration) | Migration of Caco-2 cells (%) |
|---|---|
| Control | 100.0 ± 3.8 |
| SSSR (30 µM) | 125.0 ± 2.0 ** |

**[Table 2]**

| Test compound (concentration) | Migration of Caco-2 cells (%) |
|---|---|
| Control | 100.0 ± 3.8 |
| SSSR (10 nM) | 109.7 ± 5.1 |
| FGLM-NH₂ (20 µM) | 105.9 ± 3.9 |
| SSSR (10 nM) + FGLM-NH₂ (20 µM) | 130.6 ± 0.6 ** |

### (Discussion)

From Table 1, it is apparent that SSSR trifluoroacetate at 30 µM promotes the migration of Caco-2 cells.

Further, as apparent from Table 2, a single agent of SSSR trifluoroacetate at 10 nM or a single agent of FGLM-NH₂ trifluoroacetate at 20 µM does not promote the migration of Caco-2 cells, however, when SSSR trifluoroacetate at 10 nM and FGLM-NH₂ trifluoroacetate at 20 µM are allowed to coexist, an effect of significantly promoting the migration of Caco-2 cells is observed.

### 2. Preparation Examples

General preparation examples of the therapeutic agent or prophylactic agent for a gastrointestinal disease of the present invention will be shown below.

### 1) Tablet

### Formulation 1 (in 1000 mg)

| | |
|---|---|
| SSSR trifluoroacetate | 100 mg |
| Lactose | 700 mg |
| Cornstarch | 100 mg |
| Carxboxymethyl cellulose calcium | 50 mg |
| Hydroxypropyl cellulose | 43 mg |
| Magnesium stearate | 7 mg |

A tablet having the above formulation is coated with a coating agent (for example, a coating agent such as hydroxypropylmethyl cellulose, macrogol, a silicone resin, or hypromellose phthalate), whereby a desired coated tablet is obtained. Further, by appropriately changing the amounts of the additives, a desired tablet can be obtained.

### Formulation 2 (in 1000 mg)

| | |
|---|---|
| SSSR trifluoroacetate | 1 mg |
| FGLM-NH₂ trifluoroacetate | 200 mg |
| Lactose | 600 mg |
| Cornstarch | 100 mg |
| Carxboxymethyl cellulose calcium | 50 mg |
| Hydroxypropyl cellulose | 43 mg |
| Magnesium stearate | 6 mg |

A tablet having the above formulation is coated with a coating agent (for example, a coating agent such as hydroxypropylmethyl cellulose, macrogol, a silicone resin, or hypromellose phthalate), whereby a desired coated tablet is obtained. Further, by appropriately changing the amounts of the additives, a desired tablet can be obtained.

### Formulation 3 (in 1000 mg)

| | |
|---|---|
| SSSR acetate | 0.1 mg |
| FGLM-NH₂ hydrochloride | 10 mg |
| Lactose | 650.9 mg |
| Cornstarch | 200 mg |
| Carxboxymethyl cellulose calcium | 80 mg |
| Hydroxypropyl cellulose | 50 mg |
| Magnesium stearate | 9 mg |

A tablet having the above formulation is coated with a coating agent (for example, a coating agent such as hydroxypropylmethyl cellulose, macrogol, a silicone resin, or hypromellose phthalate), whereby a desired coated tablet is obtained. Further, by appropriately changing the amounts of the additives, a desired tablet can be obtained.

### 2) Capsule

### Formulation 1 (in 150 mg)

| | |
|---|---|
| SSSR trifluoroacetate | 100 mg |
| Lactose | 50 mg |

By appropriately changing the mixing ratio of the medicament and lactose and filling an outer shell with the medicament and lactose, a desired capsule can be obtained. As the starting material of the outer shell, gelatin, hydroxypropylmethyl cellulose, or the like can be used.

### Formulation 2 (in 150 mg)

| | |
|---|---|
| SSSR trifluoroacetate | 0.1 mg |
| FGLM-NH₂ trifluoroacetate | 10 mg |
| Lactose | 139.9 mg |

By appropriately changing the mixing ratio of the medicaments and lactose and filling an outer shell with the medicaments and lactose, a desired capsule can be obtained. As the starting material of the outer shell, gelatin, hydroxypropylmethyl cellulose, or the like can be used.

### Formulation 3 (in 150 mg)

| | |
|---|---|
| SSSR acetate | 0.05 mg |
| FGLM-NH₂ hydrochloride | 10 mg |
| Lactose | 139.95 mg |

By appropriately changing the mixing ratio of the medicaments and lactose and filling an outer shell with the medicaments and lactose, a desired capsule can be obtained. As the starting material of the outer shell, gelatin, hydroxypropylmethyl cellulose, or the like can be used.

### Industrial Applicability

The present invention provides a therapeutic agent or a prophylactic agent for a gastrointestinal disease such as an ulcerative or inflammatory gastrointestinal disease, a gastrointestinal disease caused by abnormal mucosal permeability, an acquired digestion and absorption disorder caused by gastrointestinal damage due to gastrointestinal resection, radiation exposure, or a drug, or a congenital digestion and absorption disorder.

## Claims

1. A therapeutic agent or a prophylactic agent for a gastrointestinal disease, comprising a peptide having an amino acid sequence represented by Ser-Ser-Ser-Arg or a pharmaceutically acceptable salt thereof as an active ingredient.

2. A therapeutic agent or a prophylactic agent for a gastrointestinal disease, comprising the following components (a) and (b) in combination as active ingredients:
(a) a peptide having an amino acid sequence represented by Ser-Ser-Ser-Arg or a pharmaceutically acceptable salt thereof; and
(b) a peptide having an amino acid sequence represented by Phe-Gly-Leu-Met-NH₂ or a pharmaceutically acceptable salt thereof.

3. The therapeutic agent or prophylactic agent for a gastrointestinal disease according to claim 1 or 2, wherein the gastrointestinal disease is an ulcerative or inflammatory gastrointestinal disease, a gastrointestinal disease caused by abnormal mucosal permeability, an acquired digestion and absorption disorder caused by gastrointestinal damage due to gastrointestinal resection, radiation exposure, or a drug, or a congenital digestion and absorption disorder.

4. The therapeutic agent or prophylactic agent for a gastrointestinal disease according to claim 1 or 2, wherein the dosage form thereof is an oral preparation.

5. A method for treating or preventing a gastrointestinal disease, comprising administering to a patient in need thereof a therapeutically effective amount of a peptide having an amino acid sequence represented by Ser-Ser-Ser-Arg or a pharmaceutically acceptable salt thereof.

6. A method for treating or preventing a gastrointestinal disease, comprising administering to a patient in need thereof therapeutically effective amounts of the following components (a) and (b) in combination:
(a) a peptide having an amino acid sequence represented by Ser-Ser-Ser-Arg or a pharmaceutically acceptable salt thereof; and
(b) a peptide having an amino acid sequence represented by Phe-Gly-Leu-Met-NH₂ or a pharmaceutically acceptable salt thereof.

7. The method for treating or preventing a gastrointestinal disease according to claim 5 or 6, wherein the gastrointestinal disease is an ulcerative or inflammatory gastrointestinal disease, a gastrointestinal disease caused by abnormal mucosal permeability, an acquired digestion and absorption disorder caused by gastrointestinal damage due to gastrointestinal resection, radiation exposure, or a drug, or a congenital digestion and absorption disorder.

8. The method for treating or preventing a gastrointestinal disease according to claim 5 or 6, wherein the administration is performed through oral administration.

9. A peptide having an amino acid sequence represented by Ser-Ser-Ser-Arg or a pharmaceutically acceptable salt thereof for treating or preventing a gastrointestinal disease.

10. A combination of the following components (a) and (b) for treating or preventing a gastrointestinal disease:
(a) a peptide having an amino acid sequence represented by Ser-Ser-Ser-Arg or a pharmaceutically acceptable salt thereof; and
(b) a peptide having an amino acid sequence represented by Phe-Gly-Leu-Met-NH₂ or a pharmaceutically acceptable salt thereof.

11. The peptide or a salt thereof according to claim 9 or the combination of peptides or salts thereof according to claim 10, wherein the gastrointestinal disease is an ulcerative or inflammatory gastrointestinal disease, a gastrointestinal disease caused by abnormal mucosal permeability, an acquired digestion and absorption disorder caused by gastrointestinal damage due to gastrointestinal resection, radiation exposure, or a drug, or a congenital digestion and absorption disorder.

12. The peptide or a salt thereof according to claim 9 or the combination of peptides or salts thereof according to claim 10 for producing an oral therapeutic agent or prophylactic agent.


SEQUENCE LISTING

<110> YAMAGUCHI UNIVERSITY
      SANTEN PHARMACEUTICAL CO., LTD.

<120> THERAPEUTIC AGENT FOR GASTROINTESTINAL DISEASE

<130> Y 1309EU

<140> EP 10 829 941.3-2107
<141> 10.11.2010

<150> JP 2009-257569
<151> 11.11.2009

<160> 2

<210> 1
<211> 4
<212> PRT
<213> Human

<400> 1
Ser Ser Ser Arg
1

<210> 2
<211> 4
<212> PRT
<213> Human

<400> 2
Phe Gly Leu Met
1